# EUROPEAN PATENT SPECIFICATION

(11) **EP 1 196 785 B1**
(45) Date of publication and mention of the grant of the patent: **25.07.2007**
(21) Application number: 00945868.8
(22) Date of filing: 03.07.2000
(51) Int. Cl.: G01N 35/04, G02B 21/00

(54) **APPARATUS FOR TRANSPORTING CARRIERS TO A PROCESSING STATION AND METHOD OF OPERATING THE SAME**
VORRICHTUNG ZUR FÖRDERUNG VON TRÄGERN ZU EINER BEHANDLUNGSSTATION UND VERFAHREN ZU IHREM BETRIEB
DISPOSITIF SERVANT A TRANSPORTER DES SUPPORTS VERS UN POSTE DE TRAITEMENT ET SON PROCEDE DE MISE EN SERVICE

(30) Priority: 03.07.1999 EP 99202173
(43) Date of publication of application: 17.04.2002
(73) Proprietor: JANSSEN PHARMACEUTICA N.V., 2340 Beerse (BE)
(72) Inventor: NUYENS, Roger, François, Gabrielle, Armand, B-2340 Beerse (BE)
(74) Representative: Daelemans, Frank F.R.
(86) International application number: PCT/EP2000/006245
(87) International publication number: WO 2001/002867

(56) References cited:
- EP-A- 0 245 920
- WO-A-93/12430
- WO-A-97/39348
- WO-A-98/52047
- WO-A-98/59033
- DE-U- 29 712 535
- DE-U- 29 806 303
- US-A- 5 386 318

## Description

The present invention relates to handling of objects, particularly objects to be scanned optically or viewed as for instance microscope slides, specimen carriers, trays or Petri dishes. In particular, the present invention relates to an apparatus and method for transporting carriers, such as microscope slides or trays to a processing position.

### TECHNICAL BACKGROUND OF THE INVENTION

Devices for conveying specimens particularly for clinical laboratories are known from DE-U-297 12 535, WO 97/39348, WO 93/06516. However, most of these devices need to grasp the specimen, or push the specimen sideways out of a vertical magazine to a viewing position or need to move them in some other way to a viewing or processing position. It is often inconvenient grasp or push a delicate specimen holder such as a microscope slide, either because the slide is fragile, or because the specimen, or the indicia imprinted on the slide, can easily be abraded and thus obliterated. In addition, the known devices require a separate mechanism for autofocusing onto the specimen to be viewed. For example, autofocusing mechanisms are known from WO 96/01438, US 3,721,759, WO 96/10196, US 5,790,692, US 5,790,710. These require a stage for moving the specimen in at least two dimensions and often in three. This stage is in addition to the vertical delivery systems described above. Each specimen must be conveyed to a first position with one mechanism, transferred to a viewing position with another mechanism and then autofocused using yet another mechanism. This makes the combination expensive, bulky and complicated.

Transporters for single carriers have also been disclosed in documents WO98/52047, WO97/39348 and WO93/12430.

It is an object of the present invention to provide a compact system and method for delivery of specimens, particularly those to be viewed optically, to a processing or viewing position.

It is a further object of the present invention to provide an apparatus and method for obtaining an autofocus position for an optical instrument in a short time.

It is a further object of the present invention to provide an apparatus and method for obtaining an autofocus position for an optical instrument which is simple, compact and less expensive than known apparatus and methods.

### SUMMARY OF THE INVENTION

One aspect of the present invention is to integrate components of laboratory equipment, so that the components can cooperate as a compact unified, fully automated system. In one embodiment, the invention provides a transporter as defined in claim 1. Preferably, the first and second magazines and the singulator are vertically aligned so that a carrier follows a straight vertical path. This provides a compact arrangement which takes up the minimum of floor space in the laboratory. The singulator receives carriers one-at-a-time from the input magazine and moves them vertically to a processing position. After processing, the singulator then delivers each carrier to an output location where it is transferred to the output magazine. The singulator may move a carrier upwards or downwards at the processing position independent of any movement of the carriers in the input and/or output magazines. Hence, the singulator can move a carrier to a predetermined distance from either the input magazine and/or the output magazine and can adjust this position in both directions. In particular, the singulator is adapted to move a carrier very accurately and finely about the processing position, e.g. to allow autofocusing. The singulator may be described as micro-positioning, micro-stepping, micro-incrementing or micro-indexing. The drives for the input and output magazine are preferably simple gravity drives but the present invention is not limited thereto. The drive for the singulator is preferably a stepping motor or DC servo-motor. The processing position is at a location within the movement of the singulator.

The present invention may include in one embodiment a singulator comprising a release mechanism and a conveyor. The conveyor may comprise a pair of opposed belts, the belts having external protuberances or ledges. The belts are arranged vertically, so that the external ledges from the two belts define horizontally-oriented shelves which are capable of holding specimens such as carriers, slides, or other objects, in a generally horizontal orientation. The movement of the belts is preferably synchronized such that the specimens may be conveyed vertically up and down in their horizontal orientation while the belts move. The singulator is preferably adapted so that it not only transports the specimen carrier or slide to a viewing position but also provides the necessary fine vertical movements for autofocusing at the viewing position. The singulator of the present invention may also be used to transport specimens to a processing position which does not involve optical scanning buut may involve other processes.

For this purpose, the transporter in accordance with the present invention may include a processing tool which may be moved horizontally to a location above and/or below the carrier at the processing position in the singulator. Further, it is preferred if the singulator spaces the selected carrier from the output and input magazines at the processing position to allow room for the processing tool to be introduced above and/or below the selected carrier. The processing tool is preferably an optical receiver for capturing an image, e.g. a video image of the contents of the carrier. The processing tool may be any other suitable tool, e.g. a microscope or a fluorescent microscope.

The present invention includes a method of transporting specimen carriers to a processing position, comprising the steps of: providing a vertical input magazine and a vertical output magazine of carriers; singling out a carrier from the input magazine and moving it vertically to the processing position which is located between the input and output magazines; and moving the carrier to an output location where it is transferred to the output magazine.

The step of moving the carrier to the processing position is preferably carried out in such a way that there is no simultaneous movement of the carriers in the input and/or output magazines. As only one carrier is moved to the processing position at a time, the load on a drive motor is low. Further, it is preferred if the singling out and moving steps space the selected carrier from the output and input magazines to allow room for a processing tool to be introduced above and/or below the selected carrier when the carrier is at the processing position. The present invention includes moving the processing tool horizontally to a position above and/or below the carrier at the processing position and withdrawing the processing tool after carrying out processing to allow the carriers to pass. The processing tool is preferably an optical receiver for capturing an image, e.g. a video image of the contents of the carrier. The processing tool may be any other suitable tool, e.g. a microscope or a fluorescent microscope.

The dependent claims each define a separate and individual embodiment of the present invention. The present invention will now be described with reference to the following drawings.

### BRIEF DESCRIPTION OF THE DRAWINGS

Fig. 1 is schematic side-view representation of the transport apparatus according to an embodiment of the present invention.
Fig. 2 is a schematic top view of the apparatus of Fig. 1.
Figure 3 is a detail of the cam mechanism for allowing a single specimen to enter the singulator.
Fig. 4 is a side view of one type of a specimen carrier which may be used with the present invention.
Fig. 5 is a schematic cross-section of an optical receiver in accordance with the present invention.

### DETAILED DESCRIPTION OF THE ILLUSTRATIVE EMBODIMENTS

The present invention will be described with respect to certain embodiments and to certain schematic drawings but the present invention is not limited thereto but only by the claims.

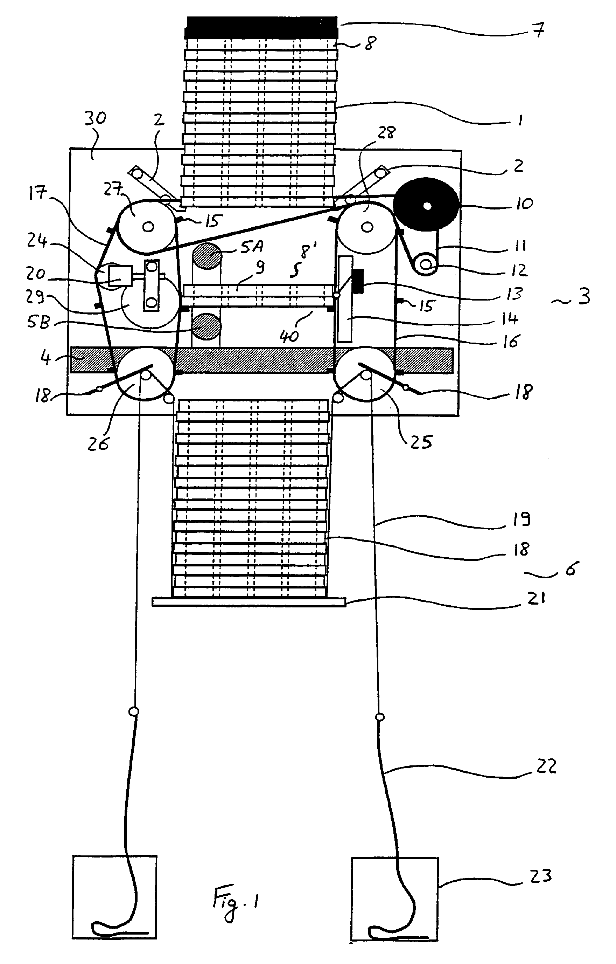

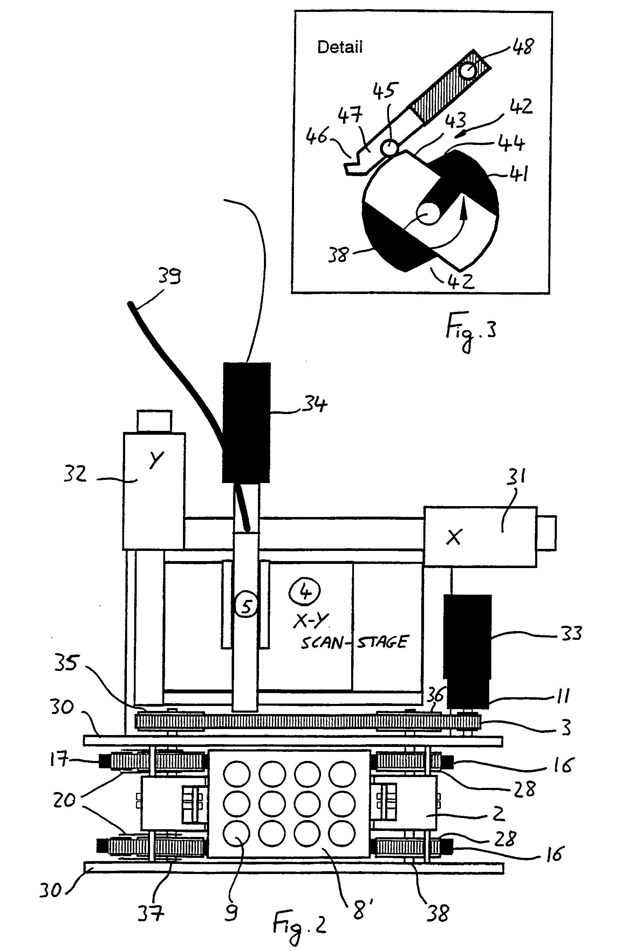

Figs. 1 and 2 show schematically a side-view (with front plate 30 removed) and a top-view (with the input magazine 1 removed) of the transporter 100 in accordance with an embodiment of the present invention. Generally, the transporter 100 includes a vertical input magazine 1 of specimen carriers 8, a singulator 3 and an output magazine 6 of carriers 8. Each carrier 8 may contain one or more wells 9 in which liquid samples, in particular, cell cultures and other biological specimens may be placed. The word carrier is meant to be interpreted in a aide sense and may include petri dishes, trays, multiwell trays, microscope slides or holders for microscope slides or any other similar carrier, tray or specimen holder which casn be stacked in an input stack and moved to a processing position. The singulator 3 receives a single carrier 8' from the input magazine 1 via cam-driven release means 2. The singulator 3 then conveys the substantially horizontal carrier 8' vertically to a processing position 40. Singulator 3 can move the carrier 8' both upwards and downwards. Due to the fact that the singulator 3 only has to move a single carrier 8, the power required for the drive for the singulator 3 is low. Also, due to the low load on this drive, high positional accuracy and fine control of movement can be obtained, e.g. by using a stepper motor or DC servo-motor drive.

As shown schematically in Figs. 1 and 2, a singulator 3 according to an embodiment of the present invention includes a release mechanism 2 and a conveyor comprising four belts 16, 17 arranged in a generally vertical orientation. Each belt 16 runs on a pair of pulleys 25, 28. Each belt 17 runs on four pulleys 24, 26, 27, 29. Pulleys 24, 29 are mechanically biased, e.g. sprung loaded, and movable to stretch belt 17 in such a way that the carrier 8 is pressed against a guide plate 14 in the processing position 40. Belts 16, 17 have a plurality of protuberances or ledges 15, located on the outer surface of each belt and projecting substantially perpendicularly to the longitudinal direction of each belt. The belts 16, 17 are preferably made of rubber or other elastomer, or a rubber-like or elastomeric material, in particular fibre reinforced rubber or elastomer or rubber impregnated or elastomer impregnated textile or of some other flexible but preferably not stretchable material. The present invention is not limited to rubber or rubber substitutes. The portions of belts 16, 17 facing each other are spaced apart and move parallel to each other. The inner distance between the belts 16, 17 is greater than the outer dimension of the carriers 8, so as to allow the carriers 8 to fit between the belts 16, 17. The distance between the protuberances 15 on the two belts 16, 17 is less than the outer dimension of the carriers 8, so that each carrier 8 can be carried by four protuberances 8, one from each of the four belts 16, 17.

The pulleys 25, 28 of belt 16 rotate in a direction opposite to that of the pulleys 24, 26, 27, 29 of belt 17. The protuberances 15 on two belts 16, 17 which face each other move in the same vertical direction. The rotation of the pair of pulleys 25, 28 is preferably synchronized with the rotation of the four pulleys 25, 26, 27, 29 so that a carrier 8 supported by the protuberances 15 remains in horizontal position during the movement. The carriers 8 may be moved either up or down by singulator 3, depending on the direction of rotation of the pulleys 24-29. Upper pulleys 27, 28 are each fixed to an axle 37, 38 respectively. Axles 37, 38 are journalled in two fixed, parallely spaced support plates 30. Axles 37, 38 are fixed at one end to pulleys 35, 36. Pulleys 35, 36 are driven by a further belt 11 which is driven by a motor 33 having a pulley 10 fixed to its output shaft. The belt 11 may be tensioned by a tensioning pulley 12 in a conventional way. Motor 33 is preferably a motor which can be driven accurately and in small increments, e.g. a stepping motor or a DC-servo-motor. Driving pulleys 35, 36 causes axles 37, 38 to rotate, hence driving pulleys 27, 28 and belts 16, 17. The backlash in the drive for belts 16, 17 should preferably be kept to a minimum by maintaining a constant positive tension in belts 16, 17 and/or to use a non-slip drive between the belts 16, 17 and the pulleys 24, 26, 27, 29, e.g. driving belts and passsing profiled pulleys. In accordance with the present invention the backlash is preferably kept below 1 mm, more preferably 500 micron or less, and most preferably 300 micron or less. The backlash is preferably cpmpensated for in the controller of the motor 33, e.g. on changes of direction the moteor is moved a set distance ,ore than specified in order to take up the backlash. The repeatability of a vertical position, i.e. the accuracy of the system is preferably better than 100 micron, more preferably 50 micron or less and most preferably 30 micron or less. 10 micron is typical. An aspect of the present invention is the provision of what may be described as a micro-incrementing, micro-positioning or micro-stepping singulator. This means that the singulator can be incremented in small steps of the order of 10 microns or less in one direction. One use of the singulator in accordance with the present invention is to bring a specimen carrier to a processing position and then to use the singulator to microindex the carrier up and down, e.g. to find a focussing position of a microscope. Thus, in accordance with the present invention, the singulator is not only a transporter for transporting a carrier from an input magazine to an output magazine, but also a singulator which can be halted at an intermediate position, moved accurately, if necessary, into a pre-determined vertical position and then can be incremented up and down about this position. All these movements can be carried out using the same motor and belt drive.

An operation of the transporter will now be described. A plurality of carriers 8 is first loaded into an input magazine 1. A weight 7 may be applied to the top of the magazine to provide a gravity drive therefor. Carriers 8 are prevented from entering the singulator 3 by a cam-driven release mechanism 2. Once allowed onto the conveyor 16,17, each carrier 8 is carried and conveyed by belts 16, 17. A carrier 8 is supported between protuberances 15 and is lowered to the processing position 40. By further movement of belts 16, 17 it is deposited into an output magazine 6.

To synchronize belts 16, 17 it is preferred if all of the pulleys 25, 26, 27, 28 have the same diameter, and are made to turn at the same rate. The pulleys 25-28 can be adjusted at the start so that the corresponding protuberances 15 on each belt combination 16, 17 are aligned horizontally. Sensor devices may be provided (not shown) to check that opposite protuberances 15 carry carriers 8 in a horizontal position. If a carrier 8 is not sufficiently horizontal, an error may cause an alarm.

The release mechanism 2 is driven in synchronism with the conveyor belts 16, 17 so that only one carrier 8 is released from the input magazine 1 onto the conveyor belts 16, 17 at a time. Fig. 3 shows a detailed schematic representation of the cam 41 and the cam follower 45 of release mechanism 2. It is advantageous to drive the cam 41 with the same motor 33 as is used to drive axles 37, 38. By this means the required synchronism between the cam 41 and the conveyor belts 16, 17 can be maintained. As shown in Fig. 2, the cam 41 is fixed to axle 38 and is located between the pulleys 28. Two release mechanisms 2 are provided opposite each other for holding and releasing carriers 8. As shown the release mechanisms 2 are placed on either side of a carrier 8 close to a pulley 27, 28 respectively. The present invention is not limited thereto. For instance, the release mechanisms 2 may be placed across the dimension of the carrier 8 which is 90° to that shown in Fig. 2.

A release mechanism 2 may include one or more (two are shown) support members 47 which are rotatably journalled on an axle 48. The release mechanism 2 also includes a cam follower 45 which bears on a cam 41 having one or more V-shaped notches 42. Each V-shaped notch 42 is bounded by sloping bearing surfaces 43, 44. Cam 41 may have a generally circular shape so that cam follower 45 remains at the same position as cam 41 rotates until a notch 42 is reached. As cam 41 rotates further cam follower 45 moves down the first sloping surface 43 and the support member 47 rotates about axle 48 making the gap between the ends of the support members 47 larger. This allows a carrier 8 to slide downwards past the ends of the support members 47. The position of the notch 42 is synchronized with the movement of belts 16, 17 so that a carrier 8' is lowered past the ends of support members 47 as protuberances 15 are at a suitable position to support this carrier 8'. As axle 38 continues to rotate the carrier 8' is lowered by movement of belts 16, 17. At the same time cam follower 45 rides up the surface 44 thus bring the end of support member 47 back to the sides of the next carrier 8 in the input magazine 1. Preferably, the relative position of the two sloping walls 43, 42 of a notch 42 may be varied so that the distance therebetween changes. For a given radius of cam 41, increasing the distance between the two sloping walls 43, 44 increases the distance that the sigulator 3 moves before the support member moves back up and supports the next carrier 8. Thus, by varying the distance between the walls 43, 44, the singulator 3 can be adapted to carriers 8 of different depths.

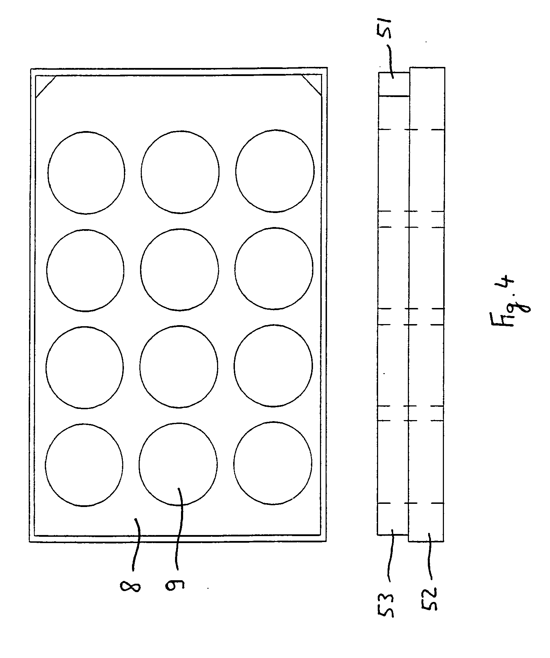

Preferably, a carrier 8 has the side-view form shown in Fig. 4 and in particular it is preferred if the carriers 8 are suitable for stacking, e.g. with a smaller upper part 51 than lower part 52. The next carrier 8 in the stack forms a lip 53. Preferably, carriers 8 are designed so that the top of one carrier seals off againts the bottom of the carrier above. This is advantageous when the carriers 8 contain liquids and evaportaion of these liquids should be prevented or reduced. As support member 47 returns to its initial position it engages with lip 53 of the next carrier 8 in the input magazine 1 and prevents this carrier from moving downwards any further at this time. The distance traveled by belts 16, 17 as cam 41 rotates from one notch 42 to the next is preferably equal to the travel of a carrier 8 from the top of the singulator to the bottom thereof, so that only one carrier 8 is present in the singulator 3 at any time. The present invention is not limited thereto. For instance two carriers 8 may be present in the singulator 3 at any one time provided these carriers 8 are adequately spaced apart.

Release of a carrier 8 from the singulator 3 to the output magazine 6 occurs automatically as belts 16, 17 travel around pulleys 25, 26. At this point the corresponding protuberances 15 move apart and allow each carrier 8 to descend into the output magazine 6. Output magazine 6 may include a plate 21 and flexible ropes 19 attached thereto. These ropes 19 run over the axles of the pulleys 25, 26 and are attached to flexible counter-weights 22 in housings 23. Movement of the ropes 19 may be enabled in one direction only by gravity and clamps 18 which press a rope 19 against the axle of the pulley 25 or 26 may prevent reverse motion. As the input magazine 6 moves down with increasing numbers of carriers 8, a longer length of counter-weight 22 is lifted up. By adjusting the weight of the flexible counter-weight 22, the additional weight of a carrier 8 in the output magazine 6 is compensated by the additional length of flexible counter-weight 22.

The transporter 100 described above is particularly useful for the delivery of carriers 8 to a optical viewing position 40 and for autofocus adjustments of the position of each carrier 8 at this position although the present invention is not limited only to optical viewing but includes additional or alternative operations such as laser cutting or trimming, addition of chemical compounds such as dies, solvents, reagents or similar, heating, cooling, drying, cutting etc. At the processing position 40 one or more tools 5 may be introduced horizontally from one or both sides of the carrier 8'. In addition tool or tools 5 may be introduced either above and/or below the carrier 8'. In particular, tool 5 may include an optical receiver 5A and a light source 5B arranged above and below the carrier 8' respectively. Optical receiver 5A and light source 5B may be attached to an XY stage 4, which can move the tool 5 in a plane XY which is perpendicular to the movement direction of carrier 8' in the singulator 3 (the Z direction). Stage 4 may be driven in the orthogonal X and Y directions by motors 31, 32, e.g. stepping motors or DC-servo motors. In operation, the tool 5 may normally be kept retracted so that it does not interfere with the operation of the singulator 3 and carriers 8. Once a carrier 8 has reached the processing position 40, the tool 5A may be moved into the carrier region using the stage 4. The present invention is not limited to only one processing position 40 but may include several, whereby the contents of carrier 8 are processed sequentially at the processing positions.

A purpose of the singulator 3 is to single out each carrier 8 to be measured and to provide sufficient distance between the singled out carrier 8' and the next carrier (stored at the bottom of the input magazine 1) so that the tool 5 may be moved into the space above and/or below the carrier 8' at the processing position 40. Preferably, sufficient space is also allowed at the processing position 40 between the singled out carrier 8' and the previous carrier 8 (stored at the top of the output magazine 6). Preferably, there is a free, unobstructed space above at least a central portion of the singled out carrier 8' which extends up to the bottom of the next carrier 8. The provision of this space is accomplished while keeping the input and output magazines 1, 6 very compact as each carrier 8 rests on the next one in these magazines. This free space is greater than the spacing of the carriers 8 in the input magazine 1 and may also be greater than the spacing between the protrusions or ledges 15 of the singulator 13. A further aspect of the singulator 3 is to provide vertical movements, both up and down, of the carrier 8' without any influence on the output magazine 6 or the input magazine 1. This allows Z direction movement of the carrier 8' to provide focusing of the contents of carrier 8' in the optical receiver 5A when the carrier is at the processing position 40.

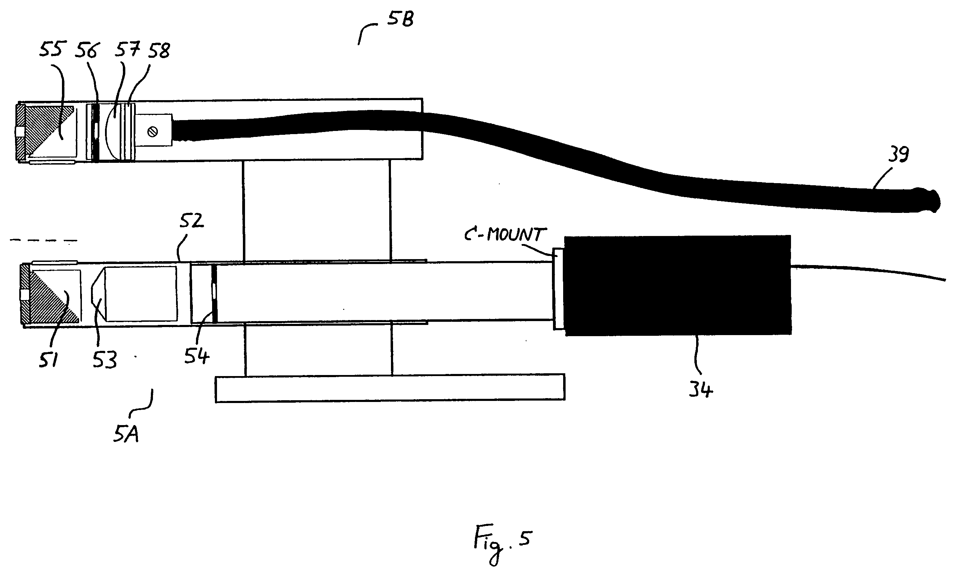

An embodiment of an optical receiver 5A and light source 5B in accordance with the present invention is shown in schematic cross-section in Fig. 5. The optical receiver 5A may include a prism 5, at its outer end remote from a camera 34 to deflect light traveling vertically from the specimen carrier 8 along tube 52 in the horizontal direction towards a camera 34. Within the tube 52 of the optical receiver 5A one or more suitable filters, lenses 53 or diaphragms 54 may be placed to direct the light from the carrier 8 to the camera 34. Camera 34 may be a video camera attached to suitable equipment for recording the video image of the specimen carrier 8, e.g. a personal computer (PC) with a video frame grabber and suitable digital storage means, e.g. a hard disk. Light for illuminating a carrier 8 may be optionally directed onto the specimen carrier 8' from above using a light source 5B including a fiber bundle 39 attached to the optical receiver 5A. Optical receiver 5A is preferably made telescopic so that its length can be adjusted to the focal length of the objective lens 53 used.

Light source 5B may also include a prism at its extreme end to direct light from along the fibre optic cable 39 to the specimen carrier 8. Further, one or more suitable diaphragms 56, condensers 57 or filters 58 may be placed between the prism 55 and the fibre optic cable 39 to direct light travelling along the fibre optic cable 39 to the prism and to control its projected spot size and colour.

Instead of being attached to an XY stage, the optical receiver 5A may be attached to an XYZ stage (not shown) so that focusing of the image received by the camera 34 may be accomplished by Z direction movements of the stage. However, it is preferred in accordance with the present invention if the Z direction movements are carried out by the singulator 3. The reason for this is that the optical receiver 5A and camera 34 are heavy and movements in particular acceleration of these parts can cause vibrations and shocks to the complete system which can disturb the focusing operations and slow these down as it is necessary to wait between each movement until vibrations have ceased in order to capture an image accurately. The carrier 8 and the conveyor belts 16, 17 are light and cause little mechanical disturbance thus allowing much more rapid focusing onto the contents of carrier 8. This accelerates automatic screening.

To improve focusing still further, a device 20 may be provided for clamping the carrier 8' at the focused position 40. Clamping device 20 (best shown in Fig. 1) may include two pulleys 24, 29 for engagement with the outer portion and inner portion of belt 17 respectively. Between the two pulleys 24, 29 is placed a small actuator which drives pulley 29 against the side of carrier 8' when suitably stimulated. The actuator may be a spring. By this means the carrier 8' is clamped between the pulley 29 and a fixed plate 14 in a steady position even when optical receiver 5A is moved sideways by stage 4, e.g. when moving from one field to another or from one well to another of the specimen carrier 8'. A position sensor 13 such as a microswitch may be used to indicate when the carrier 8 is at the processing position 40. The output of the sensor 13 may be used for activating the movement of tool 5 into the processing area.

The control of the various components of transporter 100 including the movements of stage 4, belts 16 and 17 and clamp 20 may be carried out using suitable 3-axis control devices, e.g. MultiControl 2000 supplied by Märzhauzer and a PC computer running suitable programmable software.

While the invention has been shown and described with reference to preferred embodiments, it will be understood by those skilled in the art that various changes or modifications in form and detail may be made without departing from the scope of this invention as defined in the attached claims. For example, the input magazine 1 has been described above and shown in the attached drawings as being located above the output magazine but the present invention is not limited thereto. The input magazine may be placed below the output magazine with the singulator between the two. In this case the carriers in the input magazine are driven upwards and the singulator receives a carrier and conveys it upwards towards the processing position and the output magazine. This embodiment of the present invention requires motor drives for the input and output magazines which is less preferred. Alternatively, in accordance with an embodiment of the present invention devices may be included for transporting the carrier 8 when it has arrived at the processing poistion 40 to a different processing area, e.g. by moving the carrier 8 horizontally out of the singulator 3 and optionally returing it to the singulator 3 after this processing.

The present invention has been described with reference to an input magazine and an output magazine of carriers but the present invention is not limited thereto. The carrier may be moved to the processing position and removed therefrom to some other place so that only the input magazine is generally required for the present invention. Alternatively, the carriers may no longer be required after processing at the processing position and the carrier may simply be discharged from the bottom of the singulator. For instance, the carrier may simply fall from the bottom of the singulator into a bin.

## Claims

1. A transporter (100) for transporting carriers (8) to a processing station, comprising:
a first vertical input magazine (1) of carriers (8), a second output magazine (6) of carriers (8), the first and second magazines (1, 6) being located one above the other in either order; and
a singulator (3) located between the output of the first magazine (1) and the input of the second magazine (6), the singulator (3) being adapted to receive one carrier (8, 8') at a time from the first magazine (1) and to move the carrier (8, 8') from the first magazine (1) vertically to a position (40) intermediate the first and the second magazines (1, 6) and to halt the carrier (8, 8') at this position (40).

2. The transporter according to claim 1, wherein the singulator is adapted to move a received carrier incrementally upwards or downwards at the intermediate position independent of any movement of the carriers in the first and/or second magazines.

3. The transporter according to claim 2, wherein the incremental movement comprises autofocussing about a processing position.

4. The transporter according to any previous claim wherein the singulator comprises a release mechanism (2) and a conveyor, the release mechanism (2) releasing carriers (8) from the first magazine (1) one-at-a-time to the conveyor.

5. The transporter according to any of the previous claims wherein the conveyor of the singulator comprises a pair of opposed belts, (16, 17) the belts having external protuberances or ledges (15) and the belts being arranged vertically, so that the external ledges (15) from the two belts (16, 17) define horizontally-oriented shelves which are capable of holding a received carrier in a substantially horizontal orientation.

6. The transporter according to any previous claim, further comprising a processing tool (5) movable horizontally to a location above and/or below the carrier (8, 8') when the carrier (8, 8') is at the intermediate position (40) in the singulator (3).

7. The transporter according to claim 6, wherein the processing tool (5) includes an optical receiver (5A) for capturing an image, in particular a video image of the contents of the carrier.

8. The transporter according to claim 7, wherein the optical receiver comprises a microscope.

9. The transporter according to any previous claim, wherein the carriers are Petri dishes, multi-well plates or any other device containing biological specimens.

10. A method of transporting specimen carriers (8) to a processing position (40), comprising the steps of:
providing a vertical input magazine (1) and an output magazine (6) of carriers, (8) one above the other;
singling out a carrier from the input magazine using a singulator (3) and moving it vertically to an intermediate position (40) which is located between the input and output magazines (1, 6) and halting it there; and moving the carrier to an output location where it is transferred to the output magazine (6).

11. The method according to claim 10, further comprising moving the carrier incrementally up or down at the intermediate position (40) using the singulator (3).

12. The method according to claim 11, the step of moving the carrier incrementally comprising autofocussing about a processing position.

13. The method according to any one of claims 10 to 12, further comprising the steps of:
moving a processing tool (5) horizontally to a position above and/or below the carrier at the intermediate position; and
withdrawing the processing tool after carrying out processing.

14. The method according to any of claims 10 to 13. wherein the movement of the carrier to the intermediate position is independent of movements of the carriers in the input and output magazines

15. The method according to claim 13 or 14, wherein the processing tool (5) includes an optical receiver (5A).

## Patentansprüche

1. Beförderungseinrichtung (100) zum Befördern von Trägern (8) zu einer Behandlungsstation, mit Folgendem:
einem ersten Vertikaleingabemagazin (1) für Träger (8), einem zweiten Ausgabemagazin (6) für Träger (8), wobei das erste und das zweite Magazin (1, 6) in einer beliebigen Reihenfolge übereinander angeordnet sind; und
einem Vereinzeler (3), der zwischen dem Ausgang des ersten Magazins (1) und dem Eingang des zweiten Magazins (6) angeordnet ist, wobei der Vereinzeler (3) zur Aufnahme jeweils eines Trägers (8, 8') von dem ersten Magazin (1) und zum vertikalen Bewegen des Trägers (8, 8') von dem ersten Magazin (1) in eine Position (40) zwischen dem ersten und dem zweiten Magazin (1, 6) und zum Anhalten des Trägers (8, 8') in dieser Position (40) ausgeführt ist.

2. Beförderungseinrichtung nach Anspruch 1, bei der der Vereinzeler zum schrittweisen Bewegen eines empfangenen Trägers nach oben oder nach unten in der Zwischenposition unabhängig von irgendeiner Bewegung der Träger im ersten und/oder zweiten Magazin ausgeführt ist.

3. Beförderungseinrichtung nach Anspruch 2, bei der die schrittweise Bewegung Autofokussierung um eine Behandlungsposition umfasst.

4. Beförderungseinrichtung nach einem der vorhergehenden Ansprüche, bei der der Vereinzeler einen Freigabemechanismus (2) und eine Fördereinrichtung umfasst, wobei der Freigabemechanismus (2) jeweils einen Träger (8) aus dem ersten Magazin (1) zur Fördereinrichtung freigibt.

5. Beförderungseinrichtung nach einem der vorhergehenden Ansprüche, bei der die Fördereinrichtung des Vereinzelers ein Paar einander gegenüberliegender Bänder (16, 17) umfasst, die äußere Vorsprünge oder Leisten (15) aufweisen und vertikal angeordnet sind, so dass die äußeren Leisten (15) von den beiden Bändern (16, 17) horizontal ausgerichtete Absätze definieren, die einen empfangenen Träger in einer im Wesentlichen horizontalen Ausrichtung halten können.

6. Beförderungseinrichtung nach einem der vorhergehenden Ansprüche, weiterhin mit einem Behandlungswerkzeug (5), das horizontal zu einer Position über und/oder unter dem Träger (8, 8') bewegt werden kann, wenn sich der Träger (8, 8') in der Zwischenposition (40) des Vereinzelers (3) befindet.

7. Beförderungseinrichtung nach Anspruch 6, bei der das Behandlungswerkzeug (5) einen optischen Empfänger (5A) zur Aufnahme eines Bildes, insbesondere eines Videobildes des Inhalts des Trägers, enthält.

8. Beförderungseinrichtung nach Anspruch 7, bei der der optische Empfänger ein Mikroskop umfasst.

9. Beförderungseinrichtung nach einem der vorhergehenden Ansprüche, bei der die Träger Petrischalen, Mehrfachlochplatten oder irgendwelche anderen Vorrichtungen, die biologischen Proben enthalten, sind.

10. Verfahren zum Befördern von Probenträgern (8) zu einer Behandlungsposition (40) mit den folgenden Schritten:
Bereitstellen eines Vertikaleingabemagazins (1) und eines Ausgabemagazins (6) für Träger (8) übereinander;
Vereinzeln eines Trägers von dem Eingabemagazin unter Verwendung eines Vereinzelers (3) und sein Bewegen in Vertikalrichtung zu einer Zwischenposition (40), die sich zwischen dem Eingabe- und Ausgabemagazin (1, 6) befindet, und sein Halten dort; und
Bewegen des Trägers zu einer Ausgabestelle, wo er zum Ausgabemagazin (6) übertragen wird.

11. Verfahren nach Anspruch 10, bei dem man weiterhin den Träger unter Verwendung des Vereinzelers (3) in der Zwischenposition (40) schrittweise nach oben oder nach unten bewegt.

12. Verfahren nach Anspruch 11, bei dem der Schritt des schrittweisen Bewegens des Trägers Autofokussieren um eine Behandlungsposition umfasst.

13. Verfahren nach einem der Ansprüche 10 bis 12, weiterhin mit den folgenden Schritten:
Bewegen eines Behandlungswerkzeugs (5) in Horizontalrichtung zu einer Position über und/oder unter dem Träger in der Zwischenposition; und
Zurückziehen des Behandlungswerkzeugs nach dem Durchführen der Behandlung.

14. Verfahren nach einem der Ansprüche 10 bis 13, bei dem die Bewegung des Trägers in die Zwischenposition von Bewegungen der Träger in den Eingabe- und Ausgabemagazinen unabhängig ist.

15. Verfahren nach Anspruch 13 oder 14, bei dem das Behandlungswerkzeug (5) einen optischen Empfänger (5A) umfasst.

## Revendications

1. Transporteur (100) pour transporter des supports (8) jusqu'à une station de traitement, comprenant un premier magasin d'entrée vertical (1) de supports (8), un deuxième magasin de sortie (6) de supports (8), les premier et deuxième magasins (1, 6) étant disposés l'un au-dessus de l'autre et dans n'importe quel ordre, et un dispositif d'individualisation (3) situé entre la sortie du premier magasin (1) et l'entrée du deuxième magasin (6), le dispositif d'individualisation (3) étant adapté pour recevoir un support (8, 8') à la fois à partir du premier magasin (1) et pour déplacer le support (8, 8') à partir du premier magasin (1) verticalement jusqu'à une position (40) intermédiaire entre les premier et deuxième magasins (1, 6) et pour arrêter le support (8, 8') dans cette position (40).

2. Transporteur selon la revendication 1, dans lequel le dispositif d'individualisation est adapté pour déplacer un support reçu de façon incrémentielle vers le haut ou vers le bas à la position intermédiaire indépendamment de tout déplacement des supports dans les premier et/ou deuxième magasins.

3. Transporteur selon la revendication 2, dans lequel le déplacement incrémentiel comprend une mise au point automatique autour d'une position de traitement.

4. Transporteur selon l'une quelconque des revendications précédentes, dans lequel le dispositif d'individualisation comprend un mécanisme de libération (2) et un convoyeur, le mécanisme de libération (2) relâchant les supports (8) du premier magasin (1) un par un sur le convoyeur.

5. Transporteur selon l'une quelconque des revendications précédentes, dans lequel le convoyeur du dispositif d'individualisation comprend une paire de courroies opposées (16, 17), les courroies comportant des protubérances ou des veines externes (15), et les courroies étant agencées verticalement, de telle sorte que les veines externes (15) des deux courroies (16, 17) définissent des tablettes orientées horizontalement qui sont capables de maintenir un support reçu dans une orientation sensiblement horizontale.

6. Transporteur selon l'une quelconque des revendications précédentes, comprenant en outre un outil de traitement (5) déplaçable horizontalement vers une position située au-dessus et/ou en dessous du support (8, 8') lorsque le support (8, 8') se trouve dans la position intermédiaire (40) dans le dispositif d'individualisation (3).

7. Transporteur selon la revendication 6, dans lequel l'outil de traitement (5) comprend un récepteur optique (5A) pour capturer une image, en particulier une image vidéo du contenu du support.

8. Transporteur selon la revendication 7, dans lequel le récepteur optique comprend un microscope.

9. Transporteur selon l'une quelconque des revendications précédentes, dans lequel les supports sont des boîtes de Pétri, des plaques multipuits ou n'importe quel autre dispositif contenant des spécimens biologiques.

10. Procédé de transport de supports de spécimens (8), jusqu'à une position de traitement (40), comprenant les étapes consistant à disposer un magasin d'entrée vertical (1) et un magasin de sortie (6) de supports (8) l'un au-dessus de l'autre, individualiser un support dans le magasin d'entrée à l'aide d'un dispositif d'individualisation (3) et déplacer celui-ci verticalement jusqu'à une position intermédiaire (40) qui est située entre les magasins d'entrée et de sortie (1, 6) et arrêter le support à cet endroit; et déplacer le support jusqu'à une position de sortie dans laquelle il est transféré au magasin de sortie (6).

11. Procédé selon la revendication 10, comprenant en outre le déplacement du support de façon incrémentielle vers le haut ou vers le bas à la position intermédiaire (40) en utilisant le dispositif d'individualisation (3).

12. Procédé selon la revendication 11, dans lequel l'étape de déplacement du support de façon incrémentielle comprend la mise au point automatique autour d'une position de traitement.

13. Procédé selon l'une quelconque des revendications 10 à 12, comprenant en outre les étapes consistant à déplacer un outil de traitement (5) horizontalement jusqu'à une position située au-dessus et/ou en dessous du support à la position intermédiaire; et retirer l'outil de traitement une fois le traitement exécuté.

14. Procédé selon l'une quelconque des revendications 10 à 13, dans lequel le déplacement du support vers la position intermédiaire est indépendant des déplacements des supports dans les magasins d'entrée et de sortie.

15. Procédé selon la revendication 13 ou 14, dans lequel l'outil de traitement (5) comprend un récepteur optique (5A).
